# EUROPEAN PATENT APPLICATION

(11) **EP 2 995 314 A1**
(43) Date of publication of application: **16.03.2016**
(21) Application number: 15168894.2
(22) Date of filing: 22.05.2015
(51) Int. Cl.: A61K 35/741, A61K 35/742, A61K 35/744, A61K 35/747, A61P 1/00

(54) **USE OF COLLINSELLA FOR TREATMENT OF INFLAMMATORY BOWEL DISEASE**

(30) Priority: 12.09.2014 SE 1451059
(71) Applicant: Swecure AB, 111 43 Stockholm (SE)
(72) Inventor: Saalman, Robert, 431 36 MÖLNDAL (SE); Adlerberth, Ingegerd, 413 02 GÖTEBORG (SE); Wold, Agnes, 413 10 GÖTEBORG (SE); Sjöberg, Fei, 413 24 GÖTEBORG (SE)
(74) Representative: Ström & Gulliksson AB

(57) **Abstract**

A composition comprising a carrier and a therapeutically effective amount of at least one micro-organism, the micro-organism being Collinsella, is useful in the prevention or treatment of inflammatory bowel disease in mammals.

SEQUENCE LISTING

<110> SWECURE AB

<120> USE OF COLLINSELLA FOR TREATMENT OF INFLAMMATORY BOWEL DISEASE

<130> E122920005

<140> EP15168894.2
<141> 2015-05-22

<150> SE1451059-8
<151> 2014-09-12

<160> 2

<170> BiSSAP 1.3

<210> 1
<211> 20
<212> DNA
<213> Bacteria <prokaryote>

<400> 1
agagtttgat cctggctcag                                                    20


<210> 2
<211> 17
<212> DNA
<213> Bacteria <prokaryote>

<400> 2
attaccgcgg ctgctgg                                                       17

## Description

### Field of the invention

The present invention relates to the use of micro-organism for prevention or treatment of inflammatory bowel disease, e.g. Crohn's disease or ulcerative colitis, in a mammal.

### Background

Inflammatory bowel disease (IBD) is a chronic disorder of the gastrointestinal tract. The disease is characterized by inflammation of the small intestine and/or the large intestine (colon). Symptoms of IBD include abdominal pain, persistent diarrhea, and colorectal bleeding. The cause for IBD is still elusive, but the disorder is believed to be the result of an exaggerated immune response to components of the intestine, such as intestinal microbes. Genetic predisposition, intestinal microflora composition, as well as lifestyle and environmental conditions affect the risk of developing IBD. Currently, IBD increases strongly, particularly in Western countries with good sanitary conditions and an affluent life-style. It is thought that changes in microbiota composition due to improved hygiene may be a risk factor for IBD development.

In human beings, the two main subcategories of IBD are ulcerative colitis and Crohn's disease. Both disorders cause inflammation of the digestive tract, but they differ as to the nature and location of the inflammatory reactions in the guts.

Ulcerative colitis is restricted to the colon mucosa and may involve the entire colon or only parts thereof of, such as the rectum. In contrast, Crohn's disease may affect any part of the gastrointestinal tract, i.e., from mouth to anus, although it commonly affects the lower part of the small intestine (ileum). Further, in contrast to the inflammation caused by ulcerative colitis, the inflammation caused by Crohn's disease extends deep into the intestinal wall.

Companion animals also develop IBD. Canine and feline IBD denotes a heterogeneous group of idiopathic, chronic, relapsing inflammatory disorders of the gastrointestinal tract that are immunologically-mediated. IBD can present in different forms depending on the type of inflammatory cells and the region of the gastrointestinal tract involved. The most common form of IBD involves an inflammatory infiltrate consisting of lymphocytes (lymphocytic-plasmacytic enteritis). Eosinophils may be present as the predominant cell (for example, eosinophilic gastroenteritis), but are more commonly seen as part of a mixed population of other inflammatory cells. Two less common forms of IBD are called neutrophilic and granulomatous IBD. Key clinical signs include vomiting, diarrhea and weight loss, and histopathologic lesions of inflammation may involve the stomach, small intestine, or colon.

Also horses can be affected by IBD. The IBDs in horses' compromises a collection of diseases including granulomatous enteritis (GE), lymphocytic-plasmacytic enterocolitis (LPE), multisystemic eosinophilic epitheliotropic disease (MEED), and idiopathic focal eosinophilic enterocolitis (IFEE). Disease is characterized by infiltration of the small and large intestine with inflammatory cells, including lymphocytes, plasma cells, macrophages, and eosinophils. The inflammatory condition may be limited to only a short segment of the bowel or be more diffuse. Malabsorption and a protein-losing enterocolopathy result. Diarrhea may or may not be a clinical feature.

Present treatment of IBD includes drugs, nutrition supplements, and surgery. Such treatments may be useful to control the disease by inducing or maintaining remission, or by reducing recurrence. However, there still exists a need in the art for additional therapies.

There has been suggestion in the art (cf. Berry, D. and W. Reinisch, Intestinal microbiota: a source of novel biomarkers in inflammatory bowel diseases? Best Pract Res Clin Gastroenterol, 2013. 27(1): p. 47-58; and Linskens, R.K., et al., The bacteria flora in inflammatory bowel disease: current insights in pathogenesis and the influence of antibiotics and probiotics. Scand J Gastroenterol Suppl, 2001(234): p. 29-40) that bacteria of the intestinal microbiota may be implicated in the pathogenesis of IBD. Further, there have been reports in the art, that also unaffected relatives to patient suffering from Crohn's disease may have a different composition of their microbiota compared to healthy controls (Jossens et al., Dysbiosis of the feacel microbiota in patients with Crohn's disease and their unaffected relatives. Gut, 2011, 60, 631-637).

Further, according to WO 2012/142605 there are a large number of gastro-intestinal diseases that can be, at least in part, attributable to an imbalance, disruption, or abnormal distribution of microflora in the gastrointestinal tract of a mammalian host. These diseases are stated to include, Clostridium difficile-associated diarrhea and colitis, insulin resistance, irritable bowel syndrome (IBS) or spastic colon, idiopathic ulcerative colitis, mucous colitis, collagenous colitis, Crohn's disease, inflammatory bowel disease in general, microscopic colitis, antibiotic-associated colitis, and idiopathic or simple constipation. In attempts to treating such diseases, a form of probiotics denoted fecal bacteriotherapy or fecal transfusion/transplant have been used aiming at assisting in re-establishing and/or rebalancing the gut flora (cf. e.g. WO 2013/053836). Further, studies have shown that certain gut species are more prevalent or more conserved between individuals, than others.

However, as recognized in WO 2012/142605, fecal bacteriotheraphy suffers from several disadvantages. Even if the transplant is screened for potential infectious or opportunistic microorganisms, there is always a risk that the transplant may cause an infection. Further, transplants for fecal bacteriotheraphy suffer from poor shelf life. In order to overcome the deficiencies, in WO 2012/142605 it is suggested to use a combination of pre-selected micro-organisms, which are known to be part of the normal microflora of the gut. However, no further guidance for the further selection of a proper micro-organism is provided and the suggested, but not verified, concept relies on the use of combinations of micro-organisms. As known to the skilled person, combination of different viable micro-organisms in a single formulation may affect the viability as well as the shelf-life of the formulation. Further, the teaching of WO 2012/142605 does not take the differences between various intestinal disorders, e.g. IBD and IBS (irritable bowel syndrome), and their cause into consideration. Similar to WO 2012/142605, also WO 2013/037068 discloses a complex mixture of pre-selected micro-organisms in form of a synthetic stool preparation. The synthetic stool preparation comprises bacteria isolated from a fecal sample from a healthy donor. Neither WO 2013/037068 takes the differences between various intestinal disorders, e.g. Clostridium difficile infections, IBD and IBS, and their cause into consideration.

Irritable bowel syndrome (IBS), sometimes denoted spastic colon, is a symptom-based diagnosis. IBS is condition distinct from IBD, characterized by chronic abdominal pain, discomfort, bloating, and alteration of bowel habits (diarrhea or constipation may predominate, or they may alternate). While IBD per definition is an inflammatory disease, IBS is not, though low-grade inflammation may occasionally be seen in IBS affected individuals. Diagnosis of IBS includes excluding differential diagnosis, such as Colon cancer, inflammatory bowel disease, thyroid disorders, and giardiasis. Further the Rome III criteria are often used in diagnosing IBS

### Rome III criteria

- Recurrent abdominal pain or discomfort, 3 days per month in the last 3 months, associated with two or more of the following:
   1) Improvement with defecation; and/or
   2) Onset associated with a change in frequency or stool; and/or
   3) Associated with a change in form (appearance) or stool;
- Criteria fulfilled for the last 3 months with symptom onset 6 months prior to diagnosis).

It would thus be of interest to be able to provide a single bacterial strain for targeting inflammatory bowel disease.

US 2008/003207 relates to the use of *Bacillus* or *Clostridium* for treating inflammatory bowel disease, such as ulcerative colitis (UC) and Crohn's disease (CD). The use is based on the finding that such live bacteria are effective in treating inflammatory bowel diseases. In US 2008/003207 it is stated that *Bacillus* spp. or *Clostridium* spp. may function via a mechanism including:
- modulation of the immune responses by reducing stimulating index of B cells and increasing that of T cells;
- reduction of the levels of inflammatory factors, such as IL-8 and TNF-[alpha], thus ameliorating local inflammation; and
- reduction of the levels of IgG, which are shown to be involved in the development of IBD.

However, while Bacillus is disclosed to be effective in the treatment of CD, Clostridium is disclosed to effective in the treatment of UC. At onset of IBD it may however not been known whether the patient suffers from CD or UC. It would thus be of interest to provide a microorganism targeting both diseases. Further, for risk groups it may be of interest to be able to provide prophylactic treatment to reduce the incidence of CD as well as UC.

Similar to human beings, the intestinal microbiota has also been implicated in the pathogenesis of various canine gastro-intestinal disorders including dogs with idiopathic inflammatory bowel disease (cf. Suchodolski JS, Markel ME, Garcia-Mazcorro JF, Unterer S, Heilmann RM, et al. (2012) The Fecal Microbiome in Dogs with Acute Diarrhea and Idiopathic Inflammatory Bowel Disease. PLoS ONE 7(12): e51907. doi:10.1371/journal.pone.0051907).

There is currently only one product showing clinical effect of probiotics in the treatment of canine IBD, the probiotic VSL#3 (c.f Rossi G, Pengo G, Caldin M, Palumbo Piccionello A et al. (2014) Comparison of Microbiological, Histological, and Immunomodulatory Parameters in Response to Treatment with Either Combination Therapy with Prednisone and Metronidazole or Probiotic VSL#3 Strains in Dogs with Idiopathic Inflammatory Bowel Disease PLoS One. 2014 Apr 10;9(4):e94699. doi: 10.1371/journal.pone.0094699. eCollection 2014.) VSL#3 is a multi-strain probiotic product consisting of 4 strains of Lactobacillus (*L. casei, L. plantarum. L. acidophilus, and L. delbrueckii subsp.bulgaricus*), 3 strains of Bifidobacterium (*B. longum, B. breve, and B.infantis),* and 1 strain of *Streptococcus sulivarius* subsp thermophilus. However, the amount of VSL#3 needed for proven effect was very high; dogs with IBD were given 112 to 225 x10⁹ lyophilized bacteria per 10 kg daily for 60 consecutive days. Further, as known to the skilled person, combination of different viable micro-organisms in a single formulation may affect the viability, as well as the shelf-life of the formulation. Thus, a less complex product would be desirable; especially from a regulatory perspective.

Thus, there is a need in the art for a composition comprising micro-organisms for use in prevention or treatment of inflammatory bowel disease, e.g. Crohn's disease or ulcerative colitis, in a mammal, such a human being or a dog.

### Summary of the invention

The present invention seeks to mitigate, alleviate, circumvent or eliminate at least one, such as one or more, of the above-identified deficiencies by providing a composition comprising a carrier or excipient, and a therapeutically effective amount of viable Collinsella bacteria for the use in the prevention or treatment of inflammatory bowel disease in a mammal. In the composition, at least 10% of the total number of colony forming micro-organisms are Collinsella.

According to various aspects of the invention, the composition may further comprise:
- at least one additional micro-organism, such as a micro-organism selected from the group consisting of: Sphingobacteriales, Bacillus, Enterococcus, Clostridium, Lactobacillus, Coribacteriaceae, Clostridia, Parabacteroides, and Kocuria. The additional micro-organism may be Clostridium and/or Lactobacillus; and/or
- a fiber, such as fructooligosaccharide or inulin, such as 5 to 95 wt% on dry weight basis.

Further, the composition may be characterized by:
- at least 15%, such as at least 20%, 25%, 35%, 50%, 60%, 75% or 90%, of the total number of colony forming micro-organisms in the composition being Collinsella;
- being for oral or rectal administration, such as for oral administration.
- being formulated in the form of a tablet, a capsule, a powder, or a liquid dispersion.
- containing 10⁶ CFU/g to 10¹² CFU/g of micro-organisms; and/or
- being a food product.

In addition the composition may be for prevention or treatment of Crohn's disease or ulcerative colitis, e.g. ulcerative colitis, in human beings. Furthermore, the composition may be for prevention or treatment of inflammatory bowel disease in non-human mammals, such as a dog, a cat or a horse.

Further, advantageous features of various embodiments of the invention are defined in the dependent claims and within the detailed description below.

### Description of embodiments

In attempt to provide an effective treatment of IBD, the present inventors studied the compositions of the duodenal fluid microbiota in children with new-onset inflammatory bowel disease.

This new approach is in steep contrast to previous studies, having been focused on subjects already suffering from IBD and commonly also having received treatment for their disease. The differences observed in such studies may be secondary to the disease process and/or the medication. Further, previous studies have commonly been based on the compositions of the colon microbiotia. This is not necessarily particularly relevant with respect to CD affecting the small intestine. Furthermore, as the inductive part of the mucosal immune system, the Peyer's patches, are located in the small intestine, small intestinal bacteria may exert a pronounced effect on the reaction of the immune system against gut bacteria, which may have effects on the propensity to develop IBD. The findings underlying the present invention are thus based on samples of duodenal fluid.

Further it was envisaged that it would be of importance to use a proper control group. The control group (controls), thus, consisted of subjects suspected to IBD, but later on diagnosed with a non-inflammatory, functional intestinal disorder, such as irritable bowel syndrome (IBS). Further, subjects suffering from the following diagnoses were not included in the study: allergic colitis, celiac disease, infectious colitis, unspecific colitis and indeterminate colitis. The exclusion criteria further included any prior treatment with anti-inflammatory, disease-modifying drugs or antibiotics treatment in the last three months. Previous studies have indicated that not only antibiotics, but also other drugs may affect the microbiota. By using subjects suffering from a functional intestinal disorder, not being inflammatory, as control group, bacteria not being related to inflammation are disregarded.

Based on the fecal cultivation, it could be confirmed that the control group was more frequently colonized in the colon with Bifidobacterium and Clostridium as compared to the group of children with ulcerative colitis (data not shown); thus providing basis for the treatment of IBD suggested in US 2008/003207.

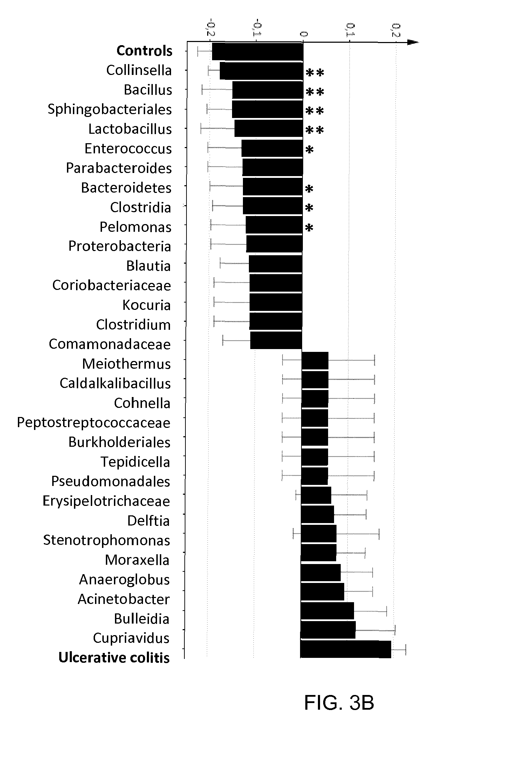

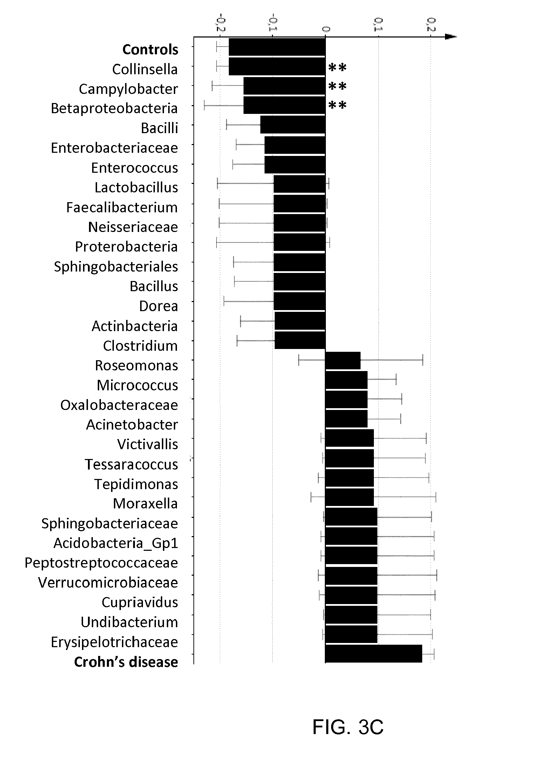

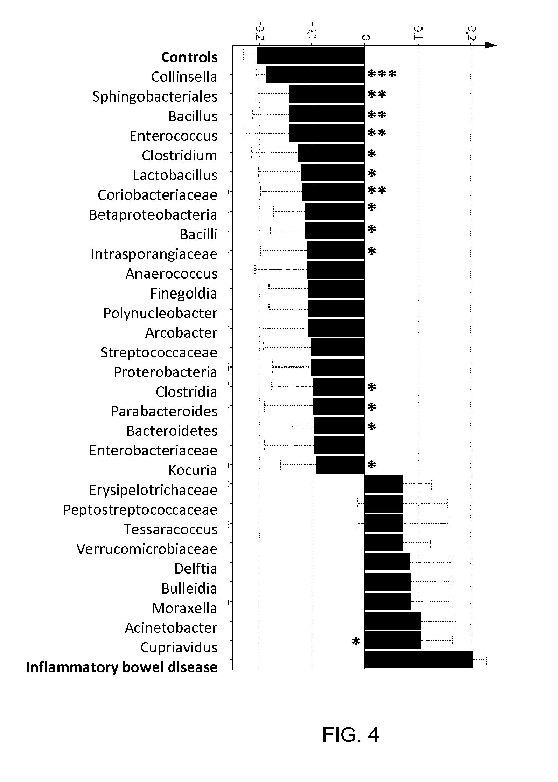

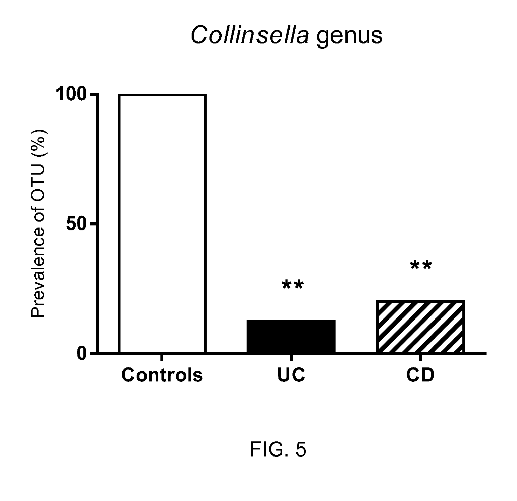

More interesting it was found that Collinsella represented the bacteria in the small intestines with the most pronounced difference between the control group and CD and UC grouped together (cf. FIG 4). Further, Collinsella was the only bacterial genus that was more frequent in the control group compared both to the CD group (cf. FIG 3C and 5), and to the UC group (cf. FIG 3B and 5), respectively. Collinsella thus represents an interesting candidate bacterium to be used in the prevention or treatment of IBD. It should be stressed that the control group consisted of subjects initially suspected to IBD, but later on diagnosed with a non-inflammatory, functional intestinal disorder, such as irritable bowel syndrome (IBS). Seemingly, a lower degree of colonization by Collinsella is not general for various intestinal disorders but specific for IBD. Collinsella is thus useful in specifically targeting IBD.

Based on the present findings it is thus concluded that Collinsella, being used in a manner similar to the one disclosed in US 2008/003207, may be used to prevent and treat CD as well as UC.

An embodiment thus relates to a composition comprising a carrier or excipient, such as a pharmaceutically acceptable one, and a therapeutically effective amount of viable Collinsella bacteria for use in the prevention or treatment of inflammatory bowel disease, e.g. Crohn's disease or ulcerative colitis, in a mammal. Similarly, another embodiment relates to a method of preventing, e.g. reducing the incidence of, or treating inflammatory bowel disease in a mammal, the method including administration of viable Collinsella bacteria. In such method, a composition comprising a carrier or excipient, such as a pharmaceutically acceptable one, and a therapeutically effective amount of viable Collinsella bacteria, is administered, in a therapeutically effective dose, to a subject in need thereof. Further, another embodiment relates to the use of viable Collinsella bacteria for the manufacture of a composition comprising a carrier or excipient, such as a pharmaceutically acceptable one, and a therapeutically effective amount of bacteria Collinsella bacteria, for the use in prevention or treatment of inflammatory bowel disease, e.g. Crohn's disease or ulcerative colitis, in a mammal. In such embodiments, the composition may comprise other microorganisms than Collinsella, but at least 10% of the total number of colony forming micro-organisms in the composition are Collinsella.

As used herein, inflammatory bowel disease, being a group of inflammatory conditions of the colon and/or small intestine, is distinct from irritable bowel syndrome (IBS).

The composition may be a pharmaceutical or nutraceutical composition. As used herein, nutraceutical composition refers to a composition that, except for its basic nutritional value, also provides extra health benefits, such as alleviating inflammatory bowel disease.

As described herein above, subjects suffering from inflammatory bowel disease have been shown to be frequently less colonized by Collinsella than control subjects. Species of Collinsella which may be used in the composition to support the healthy microbiota and thereby prevent or treat of inflammatory bowel disease include Collinsella aerofaciens, Collinsella stercoris group 1, Collinsella intestinalis group 2, and Collinsella tanakaei.

In order to be effective in preventing or treating inflammatory bowel disease, the composition should comprise a therapeutically effective amount of viable Collinsella bacteria. The actual content will depend on various factors; cf. the type of mammal, the manner of administration, and the nature and severity of the disorder. According to an embodiment, the content of Collinsella bacteria in the pharmaceutical composition is 10⁶ CFU/g to 10¹² CFU/g. Further, not only the amount of bacteria in the formulation, but also the dose administered may be of importance the treatment to be effective. A therapeutically effective dose is a dose that is sufficient to produce the desired effects in relation to the condition for which it is administered. The exact dose may be dependent on the manner of administration, nature and severity of the disorder and/or disease and the general conditions, such as age and body weight of the subject. Further, different types of mammals may, or may not, need different doses. According to an embodiment, the amount of Collinsella administered per day is within the range of 10⁵ to 10¹¹ cfu/ kg body weight. The composition may comprise other microorganisms than Collinsella. According to an embodiment, at least 15%, such as at least 20%, 25%, 35%, 50%, 60%, 75% or 90% of the total number of colony forming micro-organisms in the composition are Collinsella.

According to some embodiments, the composition is supplemented with additional beneficial bacteria also found to less frequently colonize subjects suffering from inflammatory bowel disease, than control subjects. Thus, the composition may further comprise at least one, such 1, 2, 3 or 4, additional type(s) of micro-organism selected from the group consisting of: Sphingobacteriales, Bacillus, Enterococcus, Clostridium, Lactobacillus, Coribacteriaceae, Clostridia, Parabacteroides, and Kocuria, such as from Clostridium and Lactobacillus. According to an embodiment, the composition is supplemented with Clostridium, such as Clostridium butyricum (e.g. Clostridium butyricum FERM BP-2789, also known as C. butyricum MIYAIRI 588) or Clostridium disporicum. Clostridium butyricum is used as probiotica in Asia.

In a composition specially adopted for the prevention and treatment of Crohn's disease, the pharmaceutical composition may further comprise Campylobacter.

In a composition specially adopted for the prevention and treatment of ulcerative colitis, the pharmaceutical composition may further comprise at least one, such 1, 2, 3 or 4, additional type(s) of micro-organism selected from the group constisting of: Bacillus, Sphingobacteriales, Lactobacillus, Enterococcus, bacteroides, Faecalibacterium, Subdoligranulum, Blautia, Clostridia and Pelomonas, such as from Bacillus, Sphingobacteriales, Lactobacillus, Enterococcus, bacteroides, Clostridia and Pelomonas.

Further, the composition may be supplemented with other bacteria known to be beneficial, e.g. have probiotic potential, such as one or several of Streptococci, Lactococcus, Leuconostoc, Pediococcus, Finegoldia, Ruminococcus, Roseburia and Eubacterium. According to an embodiment, the composition is supplemented with at least one additional bacteria, such 1, 2, 3 or 4 types of bacteria, selected from the group consisting of Streptococci, Lactococcus, Leuconostoc, Pediococcus, Roseburia and Eubacterium, such as from Streptococci, Lactococcus, Leuconostoc, Pediococcus. According to an embodiment, a composition supplemented with at least one beneficial with probiotic potential, is further supplemented with a bacteria also found to less frequently colonize subjects suffering from inflammatory bowel disease, than control subjects, such as Clostridium. A composition supplemented with Clostridium may further be supplemented with Lactobacillus.

In embodiments wherein Collinsella is supplemented with such other specified bacteria, Collinsella and the specified bacteria may constitute at least 50, such as at least 60%, 70%, 80%, 90%, or 95% of the total number of colony forming micro-organisms in the pharmaceutical or nutraceutical composition.

The Collinsella to be included in pharmaceutically composition, as well as any additional bacterium//bacteria, may be prepared by fermentation carried out under various conditions. If more than one type of bacteria is to be present, they may either be cultured individually or co-cultured. After the fermentation, the bacteria are collected. The collection may be performed by centrifugation. The resulting wet pellets can then be dried by any method that preserves the activity of the bacteria. As known to the skilled person, such methods include freeze drying, spray drying, heat drying and sporulation. Further, in order to maintain a high viability and protect the bacteria during the drying step, a protective agent may be added to the bacteria before the drying step. Commonly employed protective agents comprise polyhydroxy compounds, e.g. a mono-, di-, or polysaccharide, polyalcohols, e.g. sorbitol, and inorganic agents, such as calcium chloride.

In formulating the bacteria resulting from such a drying step, typically in the form of a powder, the bacteria may be mixed with a pharmaceutically acceptable carrier.

Carriers and excipients, including pharmaceutically acceptable carriers and excipients, suitable for the administration of microorganism are well-known to the skilled artisan. Such carrier should be compatible with the bacteria and not deleterious to the subject to be treated. Suitable carriers include water, microcrystalline cellulose, mannitol, glucose, defatted milk powder, polyvinylpyrrolidone, and starch, or a combination thereof.

Further, the composition may comprise a fiber, preferably a non-digestible fiber. The fiber content in the composition may be 5 to 95 wt% on dry weight basis. The composition may comprise at least 5 wt%, such as at least 10, 15, 20, 25, 30, 40, or 50 wt% fibers on dry weight basis. Further, the composition may comprise less than 95 wt%, such as less than 90, 80, 70, 60 or 50 wt% fibers on dry weight basis. Nonlimiting examples of suitable fibers include oat fibers, rye fibers, inulin, rice fibers, Psylium seed husks, soybean oligosaccharides, pectin, resistant starch, galactooligosaccharide, fructooligosaccharide (FOS), lactulose, isomaltooligosaccharides, lactitol, lactosucrose, lactulose, pyrodextrin, transgalactooligosaccharides, and xylooligosaccharides. Such fibers are known in the art to stimulate the growth and/or activity of advantageous bacteria that colonize the large bowel by acting as substrate for them. According to an embodiment, the fiber is a fructooligosaccharide or inulin.

The composition will typically be formulated for oral administration. Further, the composition may be formulated for the rectal administration. The composition can be introduced via direct access to the compromised gut (tube, enema, suppository or similar mechanism). For ulcerative colitis the composition may thus be administered rectally, e.g. by using a suppository.

Examples of suitable oral formulations of the composition include among others solid compositions, such as tablets, capsules and powders, and liquid dispersions.

In solid compositions comprising Collinsella for oral administration, the carrier(s) or excipient(s), may, without limitation, be selected from the group consisting of: corn starch, gelatin, lactose, acacia, sucrose, microcrystalline cellulose, kaolin, mannitol, dicalcium phosphate, calcium carbonate, sodium chloride, alginic acid.

Further, in providing a solid composition in form of a tablet, tablet binders such as acacia, methylcellulose, sodium carboxymethylcellulose, polyvinylpyrrolidone (Povidone3), hydroxypropyl methylcellulose, sucrose, starch, and ethylcellulose may be used. Further, also a lubricant may be used in formulating a solid composition. Examples of include magnesium stearates, stearic acid, silicone fluid, talc, waxes, oils, and colloidal silica. For tablets, also disintegrators, such as microglycolate, and alginic acid, may be present.

A solid compositions may further be formulated in manner such that the bacteria are released in the intestines, whereby avoiding exposure to the acidic conditions in the stomach. Examples, of such formulations, e.g. tablets with an enteric coating, are known in the art.

Examples of liquid compositions, e.g. dispersions, comprising Collinsella include liquid food products, e.g. nutraceutical compositions. Examples of food products include yogurt, milk, or other drinks and beverages, and a food supplement. For some applications it will not be necessary to dry the bacteria in providing the composition for administration.

Further, a nutraceutical composition may also be a composition in powder form. A composition in powder form may by dispersed in liquid, such as a beverage, before being consumed. Further, a composition in powder form may be spread over an ordinary meal. Especially, in administering the composition to non-human mammals this may be a preferred administration route. When to be used in powder form, the composition may be a freeze dried composition.

For rectal formulations the composition may be formulated as a suppository. The suppository may be hard or a soft suppository. In formulating a soft suppository, anhydrous hydropobic carriers may be used. Examples of suitable anhydrous hydropobic carriers include vegetable fat, e.g. a long-chain triglyceride, vegetable oil, e.g. a medium-chain triglyceride, and mixtures thereof. In order to provide sufficient shelf life to the composition, the water content in the formulation should be as low as possible. Thus, the water activity at 25°C of a soft suppository should preferably be less than 0.30, such as less than 0.25 or less than 0.20. Further, the water content in such a soft suppository may be less the 0.5 wt%, such as less than 0.1 wt%.

As used herein, the term medium-chain triglyceride refers to a triglyceride comprising residues of carboxylic acids comprising 6 to 12 carbon atoms. The term is intended to include separate triglycerides as well as mixtures of triglycerides. According to an embodiment, medium-chain triglycerides are C₆₋₁₂ fatty acid esters of glycerol. The fatty acid may be the same or different and they may be saturated, mono-unsaturated or polyunsaturated. A preferred example of a medium-chain triglyceride is an oil having vegetable origin.

As used herein, the term long-chain triglyceride refers to a triglyceride comprising residues of carboxylic acids comprising more than 12 carbon atoms. The term is intended to include separate triglycerides as well as mixtures of triglycerides. According to an embodiment, long-chain triglycerides are C₁₃₋₃₀, such as C₁₆₋₂₂, fatty acid esters of glycerol. The fatty acid may be the same or different and they may be saturated, mono-unsaturated or polyunsaturated. A preferred example of a long-chain triglyceride is a fat having vegetable origin.

As already described, the occurrence of inflammatory bowel disease are not limited to human beings, but also other mammals suffer from such inflammatory conditions. However, according to an embodiment, the mammal to be treated is human being. Further, in embodiments, wherein the mammal to be treated is human being, the disease to be prevented or treated may be Crohn's disease and/or ulcerative colitis, such as ulcerative colitis.

According to another embodiment, the mammal to be treated is a non-human mammal, such as a cat, a dog, or a horse. Thus, the non-human mammal may be a dog. In an embodiment, wherein the non-human mammal is a dog, the disease to be prevented or treated may be Canine idiopathic inflammatory bowel disease.

According to an embodiment "treatment " and "treating" as used herein refer to both therapeutic treatment and prophylactic or preventative measures, wherein the object is to prevent, slow down and/or lessen the disease, or repair the tissue even if the treatment is ultimately unsuccessful.

According to an embodiment, "prevent/preventing" as used herein, should not be construed to mean that a condition and/or a disease never might occur again after use of the pharmaceutical composition to achieve prevention. Further, the term should neither be construed to mean that a condition not might occur, at least to some extent, after such use to prevent said condition. Rather, "prevent/preventing" is intended to mean that the condition to be prevented, if occurring despite such use, will be less severe than without such use. Thus, prevent may be interpreted as reducing the coincidence of a given condition. Further, the term may be interpreted as prophylactic treatment.

Although the present invention has been described above with reference to specific illustrative embodiments, it is not intended to be limited to the specific form set forth herein. Any combination of the above mentioned embodiments should be appreciated as being within the scope of the invention. Rather, the invention is limited only by the accompanying claims and other embodiments than the specific above are equally possible within the scope of these appended claims.

In the claims, the term "comprises/comprising" does not exclude the presence of other species or steps. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality.

### EXPERIMENTAL

The following examples are mere examples and should by no mean be interpreted to limit the scope of the invention. Rather, the invention is limited only by the accompanying claims.

### Brief description of the drawings

These and other aspects, features and advantages of which the invention is capable of will be apparent and elucidated from the following description of embodiments of the present invention, reference being made to the accompanying drawings, in which:
FIG 1 is a graph which depicts the CFU counts of bacteria in cultured duodenal fluid from pediatric patients with symptoms suggestive of inflammatory bowel disease. Controls denote patients with symptoms, but no intestinal inflammation. Duodenal fluids were transported to the laboratory under anaerobic condition and cultured quantitatively under aerobically and anaerobically within 24h. The encircled symbols denote patients with inflammation in the duodenum;
FIG 2 depicts A) the number of reads per sample, B) the number of OTUs per sample; the encircled symbols denote patients with inflammation in the duodenum;
FIG 3 shows A) OPLS score plot showing strong clusters of the three diagnostic groups, B) OPLS loading plots depicting the microbiota comparison between controls and ulcerative colitis, C) between controls and Crohn's disease. OTUs classified above the level of genus were denoted using §Phylum, #Class, -Order and ¤Family. *P < 0.05. **P < 0.01.
FIG 4 shows the OPLS loading plots comparing controls and IBD patients (Crohn's disease and ulcerative colitis). OTUs classified above the level of genus were denoted using §Phylum, #Class, -Order and ¤Family. *P < 0.05. **P < 0.01. ***P < 0.001.
FIG 5 shows an univarite plot demonstrating the proportion of individuals within the groups having OTUs belonging to the Collinsella genus. **P < 0.01

### Materials and methods

### Patients

The study was designed to characterize the microbiota in children with IBD before diagnosis and initiation of treatment. Children 4-18 years old with suspected IBD who were referred to the Pediatric Gastroenterology Unit at the Sahlgrenska University Hospital in Gothenburg, Sweden, were asked to participate. Exclusion criteria were previous treatment with any anti-inflammatory or disease-modifying drugs, or antibiotic treatment in the previous three months. The children were hospitalized for clinical work-up and their intestinal biopsies were examined by an experienced pathologist. The diagnoses of ulcerative colitis, Crohn's disease and IBD-unclassified were based on the Porto criteria (REF). A total of 8 children (males 5, median age 15 years, range 9-16) had ulcerative colitis and 5 children (males 2, median age 11 years, range 9-16,) had Crohn's disease. Children who had symptoms compatible with IBD, but whose intestinal biopsies showed no histologic signs of inflammation and in whom IBD was excluded after extensive clinical work-up, were included as symptomatic controls (N=8, males 5, median age 15 years, range 13-16). During follow-up these patients were found to have functional intestinal disorders, mainly of temporary nature. Table 1 summarizes the characteristics and relevant clinical data of the study participants. Informed consent was obtained and the Medical Ethics' Committee of University of Gothenburg approved the study.

The children with ulcerative colitis and Crohn's disease were classified according to the Paris phenotype classification (REF) regarding localization of disease and behavior. Ulcerative colitis was extensive in all cases (E3), while the Crohn's disease was confined to the colon (L2) in one child, to the ileum and colon in 4 cases (L3) of whom one patient also had involvement of the upper gastrointestinal tract, proximal to ligamentum Treitz (L4a. All children with Crohn's disease had an inflammatory disease behavior (B1), non-stricturing and non-penetrating.

**Table 1 Clinical characterization**

| | | | | | Intestine involvement (n) | | |
|---|---|---|---|---|---|---|---|
| | **n** | **Age** | **Stools/day** | **Blood in stools** | **Pancolitis** | **Partial colon** | **Distal ileum** |
| | (total, males) | (median, range) | (median, range) | | | | |
| Crohn's disease | 5(2) | 11(9-16) | 1(0.5-2) | 1 | 3 | 1 | 4 |
| Ulcerative colitis | 8(5) | 15(9-16) | 3(1-5.5) | 7 | 8 | - | 0 |
| Control | 8(5) | 15(13-16) | 2(1-5) | 1 | 0 | 0 | 0 |

### Duodenal fluid collection and cultured

Duodenal fluid was obtained from children at esophagogastroduodenoscopy. The fluid was placed in a tube and transported to the laboratory in a gas-tight sachet in which an anaerobic and humid milieu was created using an anaerobic generator (Benex limited, County Clare, Ireland) and a piece of moistened sponge.

Anaerobiosis was confirmed upon arrival to the laboratory by controlling the anaerobic indicator included in the sachet. Aerobic and anaerobic culture was performed within 24h after sampling. In brief, 100 µl duodenal fluids were serially diluted and cultivated. For estimate of total bacterial counts, aerobic culture on Columbia blood agar and anaerobic culture on Brucella blood agar was used. The detection limit was 30 (101.52) CFU/mL. All anaerobic isolates were subcultured for purity and checked for lack of aerobic growth on subculture. Total bacterial numbers were obtained by adding the numbers of facultative and obligate anaerobic bacteria. Remaining duodenal fluid was stored frozen at -80 °C for pyrosequencing analysis.

### Pyrosequencing analysis

### DNA extraction

Bacterial DNA was extracted from 200 µl of thawed duodenal fluid according to the QIAamp DNA Stool Mini Kit protocol (Qiagen, Hildens, Germany). The DNA yield was increased by adding extra steps, in which 4 glass beads (3.0 mm) and 0.5 g zirconia beads (0.1 mm) were added to the ASL buffer suspension and homogenized at a speed of 5 m/s for 2 x 30 s using a FastPrep Cell disrupter (Savant Instruments, NY, USA), incubated at 95 °C for 5 min, and shook at 1,200 rpm (IKA vibrax Shaker, Labortechnik, Germany) for 30 min at 5 °C. DNA was quantified spectrophotometrically (v3.3 NanoDrop Technologies, Wilmington, USA).

### PCR and sequenceing library constriction

A proofreading polymerase was used for the PCR amplification of the V1-V3 region of bacterial 16S rRNA genes using the forward primer 27F (AGA GTT TGA TCC TGG CTC AG) and the reverse primer 534R (ATT ACC GCG GCT GCT GG). The PCR program was as follows: 98 °C for 30 s, followed by 25 cycles of 98 °C for 10 s, 56 °C for 30 s and 72 °C for 10 s, and a final extension at 72 °C for 1 min. The PCR products were column-purified and a second PCR was performed to add the sequencing adaptors and multiplex identifiers. The PCR condition for the second PCR was the same as above, except that steps 2 - 4 were only performed in 5 cycles. Thereafter the products were column-purified, quantified using the Qubit system and pooled. Prior to sequencing on the GS FLX, the pool of amplicons was gel purified and the DNA was recovered with a standard PCR and gel purification kit and the library quantified with the Qubit system.

### Sequence analysis,

Pyrosequencing was performed on a 454 Life Sciences Genome Sequencer FLX instrument (GATC Biotech AG in Konstanz, Germany) following standard protocols from Roche. The raw sequence reads were analyzed using the software package Quantitative Insights into Microbial Ecology (QIIME) employing default parameters for each step as described in the paper by Caporaso JG et al. (QIIME allows analysis of high-throughput community sequencing data. Nat Methods 2010, 7(5):335-336), except where specified. The raw reads were assigned to samples according to their barcodes. The singletons and possible chimeric sequences were removed before further analysis. Only high quality reads that had a minimum quality score of 25 and a read length of 200 - 500 bases were kept for further analysis. Operational Taxonomic Units (OTUs) were picked based on repetitive sequences derived from sequence clustering by software UCLUST (Edgar RC:, Search and clustering orders of magnitude faster than BLAST. Bioinformatics 2010, 26(19):2460-2461) with a minimum pairwise identity of 97%. The representative sequence of each OTU was chosen based on their abundance and length, aligned using PyNAST (a python-based implementation of NAST in QIIME). Taxonomic assignment of each representative OTU was done using the Ribosomal Database Project (RDP) classifier nomenclature method (Wang Q et al., Naive Bayesian classifier for rapid assignment of rRNA sequences into the new bacterial taxonomy. Appl Environ Microbiol 2007, 73(16):5261-5267). Finally, the abundance table of each OTU was generated from the frequency read count.

### Statistical analysis

Orthogonal Partial Least Squares (OPLS, SIMCA P+ V13, Umetrics AB, Umeå, Sweden) was used to obtain an overall pattern of the duodenal fluid microbiota in the three clinical diagnostic groups: ulcerative colitis, Crohn's disease and controls. OPLS is a regression variety of principal component analysis that finds information in the X data set that is related to a Y variable; in this case the clinical diagnosis. The presence or absence of each operational taxonomic unit, OTU were used as X variables. Univariate analyses were performed using Fisher's exacts tests (SPSS, IBM corp. NY, USA) to confirm an unequal distribution of OTUs between the diagnostic groups. The analysis was applied only to OTUs that showed the strongest contribution in the respective OPLS models. One-way ANOVA was performed to compare the overall diversity at the phylum and genus level between the diagnostic groups (Graphpad Prism 5, San Diego, USA).

### Results

Duodenal fluid was obtained during endoscopy as part of the clinical work-up in children with suspected IBD. The duodenal fluid was cultured and subjected to pyrosequencing. Microbiota composition was compared between patients with ulcerative colitis, Crohn's disease and controls, the latter representing patients referred because of symptoms suggestive of IBD, but in whom no signs of intestinal inflammation were seen in endoscopy and upon microscopic examination of intestinal biopsies. These patients were regarded as having functional gastro-intestinal disorders, generally referred to as irritable bowel syndrome.

### Quantitative culture of the duodenal fluid

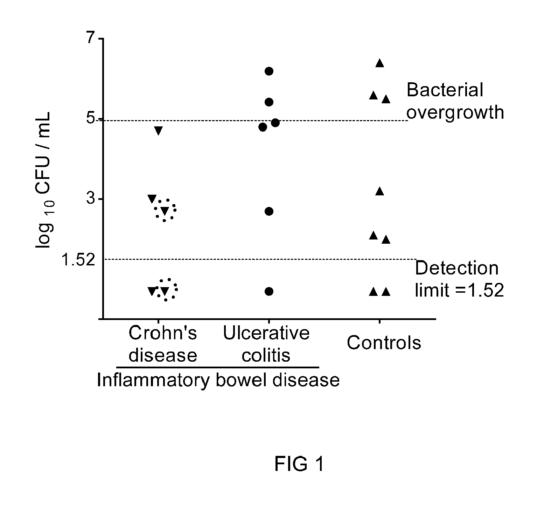

The results from aerobic and anaerobic quantitative culture are shown in FIG 1. The numbers of CFUs of facultative and anaerobic bacteria were summed. One Crohn's disease patient, several controls (3/8) and ulcerative colitis patients (4/6) had duodenal bacterial counts near or over 105/mL, the level above which a person is generally defined to have "small intestinal bacterial overgrowth". The CFU counts did not differ statistically between the clinical groups (One-way ANOVA). Two patients with Crohn's disease suffered from duodenitis; both had low bacterial counts in small intestinal fluid: 102.7CFU/mL and below the detection limit (30 bacteria/mL, 101.52), respectively.

### NGS dataset analysis and OTU assignment

DNA was extracted from the duodenal fluids and the V1-V3 variable regions of the bacterial 16S rRNA gene was amplified by PCR and pyrosequenced. Altogether a dataset of near 700,000 high quality sequences were generated and 99% of the reads had an average read length of 495 (±24) nucleotides and a mean of 32,000 (±1.2 k) sequence reads per sample.

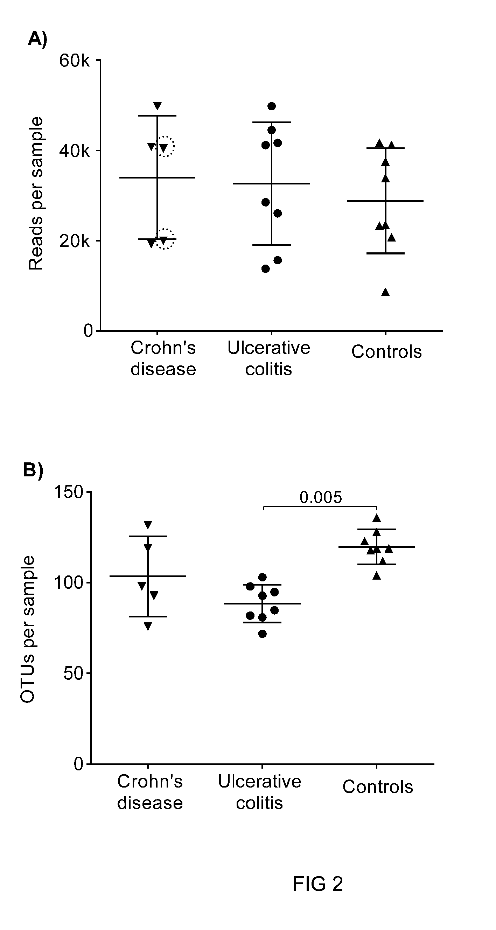

The number of reads per sample did not differ significantly between the diagnostic groups (FIG 2A). However, duodenal fluids yielding > 104 CFU/mL based on culture generated more sequence reads than fluids with <104/viable bacterial/mL (mean: 40 k/sample vs. 25 k/sample, P = .007). Fluids from the two patients with Crohn's disease manifesting as duodenitis yielded 40 k and 20 k reads, respectively.

Sequences were assigned to genus-level OTUs using a 97% pairwise identity cut-off. A total of 322 OTUs were detected in the dataset. The number of OTUs per patient was calculated. As seen in FIG 2B, patients with ulcerative colitis had fewer OTUs per sample (average 90) than controls (average 120, P = .005). Patients with Crohn's disease had a wide span of OTU numbers. The average number of OTUs per patient was equally high in those with >104 CFU/mL of viable bacteria as in those with <104 CFU/mL (107±21 OTUs vs. 103±19 OTUs).

### Phylum distribution

A total of 19 bacterial phyla were represented in the dataset. The phylum distributions of the sequences were as follow: Firmicutes (56 %), Proteobacteria (17 %), Actinobateria (11 %) and Bacteroidetes (6 %). The rest of 15 phyla comprised 5 % of the sequences and 5% of all sequences (29 k reads) could only be classified as bacteria. At the genus level, Streptococcus and Veillonella, belonging to the phylum Firmicutes, were the most prevalent, comprising of 31 % and 11 % of the total number of reads, respectively.

### Duodenal microbiota: classification to the genus level

Most OTUs (232, i.e. 72%) could be assigned a genus, another 59 (18%) of the OTUs could only be classified to the family level, 17 to the order level, 11 to the class level and 3 OTUs only to the phylum level. We compared the overall bacterial composition between the three diagnostic groups using the multivariate pattern recognition method OPLS. In each patient, the presence or absence of each of the total 322 OTUs identified in the patient material was recorded and the patterns were compared between the clinical groups.

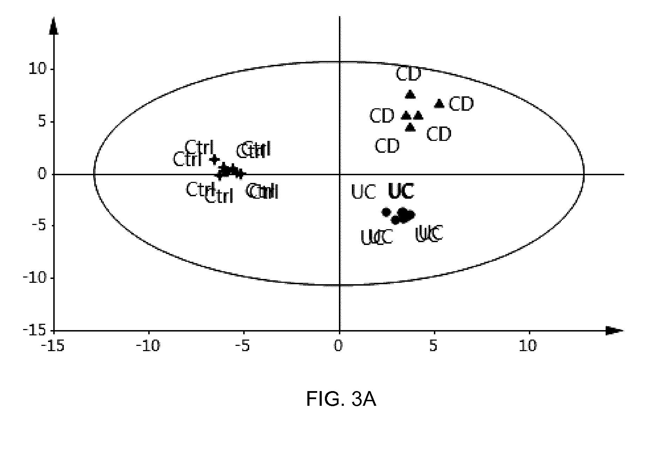

As seen in FIG 3A, patients with ulcerative colitis and patients with Crohn's disease could readily be separated from one another and from the controls. The loading plots show which OTUs that contributed to separating controls and ulcerative colitis (FIG 3B), or controls and Crohn's disease (FIG 3C), respectively. The larger the bar, the stronger the contribution of the variable to the model; error bars denote the variability of the data. Bars pointing in the same direction as the diagnosis bar are positively associated with that diagnosis. For clarity, OTUs contributing little to the model were eliminated using the variable importance for the projection (VIP) approach using a threshold VIP-value of 1.5. The OTUs yielding the largest contribution to the model were confirmed by univariate analysis (asterisks above the column bars). We investigated the microbiota differences at the genus OTU level (or the nearest classifiable level, i.e. family, order, class or phylum). It may be seen that the separation between patients and controls was mainly achieved by the increased prevalence of a number of genera in the control group that were less prevalent in the IBD groups. Thus, when the microbiota was compared, by univariate data analysis, between controls and patients with ulcerative colitis, controls had significantly higher prevalence of OTUs classified to the genera Collinsella (FIG 3C and 5, P = .001), Lactobacillus (P = .007), Bacillus (P = .007) and to the order Sphingobacteriales (P = .007) (FIG 3C). Other OTUs that were more common in controls than in ulcerative colitis patients included the genera Parabacteroides (P =.04), Pelomonas (P = .04) and Enterococcus (P = .03), an unclassified OTU belonging to the class Clostridia (P = .04) and another belonging to the phylum Bacteroidetes (P = .04), but, considering the large numbers of comparisons, these differences should be cautiously interpreted. Interestingly, not a single OTU was significantly more prevalent in the ulcerative colitis patients than in controls, confirming the greater average number of OTUs in the control group (FIG 2B) and suggesting that absence of a range of bacteria is the characteristic of ulcerative colitis, rather than the presence of particular disease-provoking bacteria.

When comparing patients with Crohn's disease and controls, Colinsella was also less prevalent in the patients, in accordance to their reduced prevalence among patients with ulcerative colitis (FIG 3C and 5). In addition, Campylobacter (P = .007) and an unknown member of the Betaproteobacteria class (P = .007) (FIG 3C) were also less prevalent in pediatric patients with Crohn's disease than in controls.

As several OTUs seemed to be less common in both ulcerative colitis and Crohn's disease patients, than in controls (see Fig 3B and Fig 3C), we also compared the duodenal microbiota of IBD patients as a combined group, with that of controls. As seen in FIG 4, the results were similar as above but a greater statistical significance level was achieved. Hence, children with IBD were significantly less often colonized with Collinsella (P = .000 ∞), Bacillus (P = .003), Enterococcus (P = .003), Lactobacillus (P = .007), one OTU belonging to the order Sphingobacteriales (P = .003), as well as several other OTUs, compared to controls (FIG 3C).

### Summary

Our controls showed the anticipated typical healthy small intestinal flora dominated by genera such as Lactobacillus, Enterococcus and Streptococcaceae and comprising members of all four dominating intestinal phyla. With the sequence-based analysis, we could demonstrate that the microbiota of control children had a higher diversity across all phyla and also contained more OTUs that could not be assigned to any known bacterial phylum or even be classified as bacteria, than the microbiota of children with ulcerative colitis. The microbiota of children with Crohn's disease also tended to be less complex that of controls, but the variation was larger in the Crohn's patient group and the differences did not reach significance. Clearly, however, the microbiota of children with IBD particularly ulcerative colitis is more restricted than that of children without IBD.

Quantitative culture revealed bacterial levels exceeding 105 colony forming units/mL in 26% of the children. Pyrosequencing analysis showed that the small intestinal microbiota of children with ulcerative colitis was less diverse i.e. contained fewer operational taxonomic units (OTUs), than that of controls, including fewer OTUs within the Actinobacteria, Bacteroidetes and Firmicutes phyla. Several bacterial genera were less often isolated from children with ulcerative colitis than from controls, including Collinsella (P < .000), which was also Bacillus (P = .003), Enterococcus (P = .003), Lactobacillus (P = .007), unclassified Sphingobacteriales (P = .003) and Bacilli (P = .007). Collinsella was also less common in Crohn's disease patients compared to controls.

In conclusion, the duodenal microbiota of children with new-onset IBD, who had neither received antibiotics, nor disease-modulation drugs, was distinctly different from that of control children displaying similar symptoms but no intestinal inflammation. Particularly, lower diversity characterized the duodenal microbiota in children with IBD.

Thus, the data supports the use of Collinsella in the prevention or treatment of inflammatory bowel disease in a mammal.

## Claims

1. A composition comprising a carrier or excipient, and a therapeutically effective amount of viable Collinsella bacteria for the use in the prevention or treatment of inflammatory bowel disease in a mammal, wherein least 10% of the total number of colony forming micro-organisms in the composition are Collinsella.

2. The composition according to claim 1, wherein the composition further comprises at least one additional micro-organism selected from the group consisting of: Sphingobacteriales, Bacillus, Enterococcus, Clostridium, Lactobacillus, Coribacteriaceae, Clostridia, Parabacteroides, and Kocuria.

3. The composition according to claim 2, wherein said additional micro-organism(s) are selected from the group consisting of: Clostridium and Lactobacillus.

4. The composition according to any one of the claims 1 to 3, wherein least 15%, such as at least 20%, 25%, 35%, 50%, 60%, 75% or 90%, of the total number of colony forming micro-organisms in the composition are Collinsella.

5. The composition according to claim 4, wherein
- the composition further comprises at least one additional micro-organism selected from the group consisting of: Sphingobacteriales, Bacillus, Enterococcus, Clostridium, Lactobacillus, Coribacteriaceae, Clostridia, Parabacteroides, and Kocuria; preferably from the group consisting of: Clostridium and Lactobacillus; and
- Collinsella and the specified additional micro-organism constitutes at least 50, such as at least 60%, 70%, 80%, 90%, or 95%, of the total number of colony forming micro-organisms in the composition.

6. The composition according to any one of the claims 1 to 5, wherein the composition is a composition for oral or rectal administration, such as for oral administration.

7. The composition according to claim 6, wherein the composition is for oral administration and formulated in the form of a tablet, a capsule, a powder, or a liquid dispersion.

8. The composition according any one of the claims 1 to 7, wherein the composition comprises a fiber, such as fructooligosaccharide or inulin.

9. The composition according to any one of the claims 1 to 8, wherein the amount of the administered micro-organism(s) is within the range of 10⁶ to 10¹² cfu per day.

10. The composition according to any one of the claims 1 to 9, wherein the pharmaceutical composition contains 10⁶ CFU/g to 10¹² CFU/g of the micro-organism(s).

11. The composition according to any one of the claims 1 to 10, wherein the composition is a food product.

12. The composition according to any one of the claims 1 to 11, wherein the mammal is a human being and the inflammatory bowel disease is Crohn's disease or ulcerative colitis, such as ulcerative colitis.

13. The composition according to any one of the claims 1 to 11, wherein the mammal is a non-human mammal, such as a dog, a cat or a horse.

14. The composition according to claim 13, wherein the non-human mammal is a dog.

15. The composition according to claim 14, wherein the inflammatory bowel disease is Canine idiopathic inflammatory bowel disease.
